(19) Europäisches Patentamt
European Patent Office
Office européen des brevets

(11) EP 1 633 394 B1

(12) **EUROPÄISCHE PATENTSCHRIFT**

(45) Veröffentlichungstag und Bekanntmachung des Hinweises auf die Patenterteilung:
**03.01.2007 Patentblatt 2007/01**

(21) Anmeldenummer: **04739811.0**

(22) Anmeldetag: **11.06.2004**

(51) Int Cl.:
***A61K 38/50*** *(2006.01)* ***A61P 35/00*** *(2006.01)*

(86) Internationale Anmeldenummer:
**PCT/EP2004/006320**

(87) Internationale Veröffentlichungsnummer:
**WO 2004/108153 (16.12.2004 Gazette 2004/51)**

(54) **PHARMAZEUTISCHE KOMBINATIONSPRÄPARATE ZUR KREBSTHERAPIE ENTHALTEND GLUTAMINASE UND ANTINEOPLASTISCHE ANTHRACYCLINE ODER PLATINVERBINDUNGEN**

COMBINED PHARMACEUTICAL PREPARATIONS FOR THE TREATMENT OF CANCER, CONTAINING GLUTAMINASE AND ANTINEOPLASTIC ANTHRACYCLINES OR PLATINUM COMPOUNDS

PREPARATIONS PHARMACEUTIQUES COMBINEES POUR TRAITER LE CANCER, CONTENANT DE LA GLUTAMINASE ET DE L'ANTHRACYCLINE ANTI-NEOPLASTIQUE OU DES COMPOSES DE PLATINE

(84) Benannte Vertragsstaaten:
**AT BE BG CH CY CZ DE DK EE ES FI FR GB GR HU IE IT LI LU MC NL PL PT RO SE SI SK TR**

(30) Priorität: **11.06.2003 DE 10326821**

(43) Veröffentlichungstag der Anmeldung:
**15.03.2006 Patentblatt 2006/11**

(73) Patentinhaber: **UNIVERSITY OF SOUTH CAROLINA**
**Columbia, SC 29208 (US)**

(72) Erfinder:
- **LEENDERS, Frank**
  **13187 Berlin (DE)**
- **SEIFERT, Wenke**
  **10439 Berlin (DE)**

(74) Vertreter: **Dey, Michael et al**
**Weickmann & Weickmann,**
**Postfach 860 820**
**81635 München (DE)**

(56) Entgegenhaltungen:
**DE-A- 4 140 003**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 633 394 B1

**Beschreibung**

Die Erfindung betrifft pharmazeutische Kombinationspräparate, die das abnormale Wachstum von Tumorzellen inhibieren. Diese Kombinationspräparate umfassen als Wirkstoffe Verbindungen, die Glutaminase-Aktivität besitzen, in Kombination mit bestimmten Antineoplastika. Insbesondere betrifft die Erfindung Kombinationspräparate von Glutaminase-Aktivität aufweisenden Verbindungen mit cytostatisch wirksamen Verbindungen gemäß den Ansprüchen.

Unter dem Oberbegriff Krebs ist eine Vielzahl verschiedener bösartiger (maligner) Erkrankungen zusammengefasst, die sich dadurch auszeichnen, dass Zellen unkontrolliert wachsen, eine Zelldifferenzierung fehlt und benachbarte Gewebe durchdrungen sowie Metastasen gebildet werden. Nahezu jedes Gewebe kann Ursprung für eine solche maligne Erkrankung sein.

Heutige Standard-Krebstherapien mit antineoplastischen Wirkstoffen bergen trotz der fortgeschrittenen Entwicklung erhebliche Nachteile und Risiken für den Patienten. Aufgrund ihres unspezifischen antiproliferativen Effekts und der hohen Dosierung werden durch diese Antineoplastika nicht nur Tumorzellen, sondern auch gesunde, schnell wachsende Zellen geschädigt, wie z.B. Schleimhäute, Zellen des blutbildenden Systems (Knochenmark) und Haarfolikel. Die Behandlung mit Antineoplastika ist daher meist mit starken Nebenwirkungen verbunden, die das allgemeine Wohlbefinden von Patienten beeinträchtigen (akute Nebenwirkungen), zu irreversiblen Schädigungen von gesundem Gewebe führen und das Risiko von Zweittumoren erhöhen. Ferner können Tumore Resistenzen gegen Wirkstoffe ausbilden, was bei MehrfachAnwendungen an einem Patienten zum Wirkungsverlust führt.

Zur Erzielung besserer Wirksamkeiten und Verminderung der Resistenzbildung werden häufig mehrere Wirkstoffe kombiniert und gleichzeitig zur Therapie eingesetzt (Polychemotherapie). Trotz dieser Strategie sind die oben beschriebenen Probleme bisher nicht zufriedenstellend gelöst. Es ist aus wirtschaftlicher und medizinischer Sicht daher dringend erforderlich, neue und schonende Therapien für die Krebsbekämpfung zu finden.

Ein möglicher Ansatz zur Therapie solcher maligner Erkrankungen ist die Reduktion der Glutamin-Konzentration im Blutkreislauf. Glutamin. ist die häufigste Aminosäure im Blutkreislauf und spielt als Stickstoff- und Energiequelle sowie als Basiskomponente für viele zelleigene Synthesen eine große Rolle. Insbesondere Tumorzellen sind aufgrund ihres starken Wachstums auf Glutamin aus dem Blutkreislauf angewiesen.

In den 1980er Jahren wurden zahlreiche Ansätze verfolgt, Glutamin spaltende Enzyme oder reaktive Glutaminanaloga zur Krebstherapie einzusetzen, die den Tumoren das benötigte Glutamin entziehen. Roberts et al. zeigten, dass Pseudomonas 7A Glutaminase-Asparaginase eine antineoplastische Aktivität gegen eine Vielzahl von Leukäimie-Erkrankungen bei Nagem, gegen Ascites-Tumoren und bestimmte solide Tumore besitzt (DE 41 40 003 A1 und WO 94/13817 A1). Zusätzlich wurde in Tierexperimenten mit athymischen Mäusen ermittelt, dass die Kombination von Glutamin-Analoga (z.B. 6-diazo-5-oxo-L-Norleucin (DON)) und Glutaminase stark inhibierend auf menschliche Kolon-, Brust- und Lungenkarzinome wirkt (McGregor, W & Roberts, J (1989): Proc. Amer. Assoc. Cancer Res. 30, 578). Ferner wurde gezeigt, dass die Behandlung mit Glutaminase die Resistenzbildung gegen Methotrexat verzögert (Roberts, J., Schmid, F.A. & Rosenfeld, H.J. (1979): Cancer Treat. Rep.63: 1045 - 1054).

Die anfänglich vielversprechenden Tierversuche führten jedoch noch nicht zu vermarktbaren Medikamenten, da alle Therapieansätze mit Glutaminase oder Glutaminanaloga (z.B. DON, Acivicin) zunächst wegen zu starker toxischer Nebenwirkungen abgebrochen werden mussten (Medina MA (2001), Glutamin and cancer, The Journal of Nutrition, Vol 131 (9): 2539S - 42S). Trotz des idealen Wirkprinzips einer Glutamin-Depletion-Therapie hat sich bisher keine Therapie auf der Basis von Proteinen mit Glutaminase-Aktivität durchsetzen können.

Da jedoch bis heute Krebserkrankungen nur unzureichend behandelt werden können, wäre es von größter medizinischer und wirtschaftlicher Bedeutung, einen Weg zu finden, wie man den vielversprechenden Ansatz einer Glutamin-Depletion-Therapie in Zukunft nutzen kann.

Der vorliegenden Erfindung lag deshalb die Aufgabe zugrunde, die Wirkung von Antineoplastika zu steigern und Präparate bereitzustellen, die in Konzentrationen eingesetzt werden können, die keine bzw. geringe Toxizitäten und Antikörperbildung hervorrufen.

Überraschend wurde gefunden, dass bestimmte an sich bekannte Antineoplastika in Kombination mit Verbindungen mit Glutaminase-Aktivität geignet sind, diese Aufgabe zu lösen. Die Kombinationen wirken synergistisch, direkt oder indirekt toxisch auf sich teilende Zellen und können somit zur antineoplastischen Therapie eingesetzt werden. Die Glutaminase-Aktivität aufweisende Komponente dient als Verstärker, der die erforderliche Dosis von Antineoplastika senkt und die Nebenwirkungen sowie die Spätfolgen reduziert. Als Antineoplastika werden Platin-Komplexe, insbesondere cis-Platin, Oxaliplatin, Carboplatin oder Derivate davon oder Anthracycline, insbesondere Doxorubicin oder Daunomycin oder Derivate davon eingesetzt.

Insbesondere betrifft die Erfindung Kombinationspräparate von Verbindungen mit Gtutaminase-Aktivitäit und cytostatisch wirksamen Verbindungen gemäß den Ansprüchen Cytostatika sind in der antineoplastischen Therapie schon seit längerer Zeit ein anerkanntes und weit verbreitetes Behandlungsprinzip.

Sie werden eingesetzt, um maligne Zellen mit ungehemmtem Wachstumsverhalten zu zerstören. Normale und gesunde Zellen sollen möglichst wenig geschädigt werden.

Überraschenderweise wurde gefunden, dass Verbindungen, die GlutaminaseAktivität besitzen, in Kombination mit Antineoplastika eine synergistische Wirkung ausüben. So wurde im Falle der Anwendung der erfindungsgemäß eingesetzten Cytostatika festgestellt, dass eine Verstärkung des antiproliferativen oder antitumoralen Effekts auftritt.

Im Sinne der vorliegenden Erfindung werden unter Verbindungen, die Glutaminase-Aktivität besitzen, die Proteine bzw. Enzyme Glutaminase; Glutaminase-Asparaginase; Glutaminase-Analoga; Derivate und Modifizierungen verstanden, die entweder auf natürliche Weise vorkommen oder synthetisch hergestellt werden und die die Glutaminproduktion hemmen. In einer Ausführungsvariante können die Verbindungen modifiziert oder mit Schutzsubstanzen versehen sein. Bevorzugt werden Polyethylenglykol modifizierte Verbindungen eingesetzt. Besonders bevorzugt wird gentechnisch hergestellte Glutaminase verwendet oder/und Pseudomonas-Glutaminase. Erfindungsgemäß bevorzugt einsetzbare Glutaminasen sind in WO 94/13817 beschrieben.

Gegenstand der vorliegenden Erfindung ist neben den Kombinationspräparaten die Verwendung von Antineoplastika gemäß den Ansprüchen mit Verbindungen, die Glutaminase-Aktivität besitzen, zur Behandlung von Krebs und anderen Krankheiten, die im Zusammenhang mit abnormaler Zellproliferation stehen.

Insbesondere bestehen solche Wirkstoffkombinationen aus einer GlutaminaseAsparaginase, vorzugsweise Pseudomonas 7A Glutaminase-Asparaginase, und einem oder mehreren Antineoplastika aus den o.g. Gruppen.

Kombinationen dieser Erfindung zeichnen sich dadurch aus, dass die antitumorale Wirkung der erfindungsgemäß verwendeten Antineoplastika durch Kombination mit einer Glutaminase-Aktivität aufweisenden Verbindung signifikant verstärkt wird und der Proteinwirkstoff selber in Konzentrationen eingesetzt werden kann, die keine toxischen Wirkungen hervorrufen.

Erfindungsgemäß können die Kombinationspräparate für die Krebstherapie eingesetzt werden. Es können insbesondere subtherapeutische Dosen einer Verbindung mit Glutaminase-Aktivität und die erfindungsgemäßen Antineoplastika verwendet werden, die synergistisch das Tumorzellwachstum inhibieren. Das bedeutet, dass die Kombination der Wirkstoffe eine wesentlich größere antineoplastische Aktivität aufweist als jeweils ein Wirkstoff dieser Wirkstoffklasse alleine.

Zu den antineoplastischen Wirkstoffen, die sich für eine Kombination eignen, gehören erfindungsgemäß Platin-Komplexe, insbesondere cis-Platin Oxaliplatin, Carboplatin oder Derivate davon, sowie Anthracycline, z.B. Actinomycin D, Mitoxantron, und insbesondere die DNS-Interkalatoren Doxorubicin und Daunomycin (Daunorubicin).

Die Anwendung einer Kombinationstherapie mithilfe der pharmazeutischen Präparate der vorliegenden Erfindung bietet den Vorteil der synergistischen Verstärkung der antitumoralen Wirksamkeit der Einzelsubstanzen. Dadurch ergibt sich ferner die Möglichkeit der Reduktion der Dosen und damit der Toxizitäten der Einzelsubstanzen bei gleichzeitiger Erhaltung der antitumoralen Wirksamkeit bei Kombination der Einzelsubstanzen. Eine Kombinationstherapie der o.g. Einzeltherapieprinzipien bietet ferner die Möglichkeit der Überwindung von Cytostatika-Resistenzen, wobei sowohl Substanzgruppenresistenzen als auch Mehrfachresistenzen (pleiotrope Cytostatikaresistenz) in Frage kommen.

Ohne an eine Theorie gebunden sein zu wollen, wird vermutet, dass der erfindungsgemäß beobachtete synergistische Effekt auf folgendem Wirkmechanismus beruht. Alkylierende Cytostatika, wie z.B. Platinpräparate, üben eine Wirkung auf Krebszellen insbesondere während der Zellteilung aus. Über die Energieverarmung durch Glutaminentzug mittels Glutaminase sowie durch das Fehlen von Glutamin bei der DNS-Synthese durch die Glutaminase, kommt es zu einer Verlängerung der Zellteilungszeit und somit zu einer Verlängerung der vulnerablen Phase der Krebszellen für alkylierende Substanzen. Wie bekannt ist (beispielsweise aus Gewebekulturuntersuchungen), wachsen die Krebszellen im Medium erst ab einer gewissen Konzentration von Glutamin an. Weiterhin wurde mittels Durchflusscytometrie festgestellt, dass die akute myeloische Leukämie besonders in den frühen Morgenstunden auf cytostatische Therapien anschlägt. Zusätzlich fehlt der Krebszelle durch den Glutaminentzug in der Zelle wie auch außerhalb ein wichtiges Antioxidans, was weiter zur Vulnerabilität durch Cytostatika beiträgt.

Bei der Anwendung der Kombinationstherapie ist es möglich, die Wirkstoffe in einer sogenannten fixen Kombination, d.h. in einer einzigen pharmazeutischen Formulierung zu verabreichen, in der beide Wirkstoffe enthalten sind, oder eine sogenannte freie Kombination zu wählen, bei der die Wirkstoffe in Form von getrennten pharmazeutischen Formulierungen gleichzeitig oder aber auch nacheinander appliziert werden können.

Sind die Wirkstoffe Feststoffe, so können die Wirkstoffe nach üblichen Verfahren zu festen Arzneimittelpräparaten verarbeitet werden, indem man z.B. beide Wirkstoffe miteinander vermischt und mit üblichen Träger- oder Hilfsstoffen zusammen beispielsweise zu Tabletten verpresst. Es ist aber auch möglich, die Wirkstoffe getrennt voneinander in einer verkaufsfertigen Verpakkungseinheit zur Verfügung zu stellen, wobei die Verpakkungseinheit die beiden Wirkstoffe in getrennten pharmazeutischen Formulierungen enthält.

Werden die Wirkstoffe in Form von Injektionslösungen zur Verfügung gestellt, so können diese die in Frage kommenden Wirkstoffkombinationen bereits in fertig injizierbarer gelöster Form enthalten. Prinzipiell ist es aber auch möglich, je eine parenterale Formulierung für jeden in Frage kommenden Wirkstoff in einer Verpakkungseinheit zur Verfügung zu stellen, sodass die Injektionslösungen gegebenenfalls getrennt voneinander appliziert werden können. Bei Unverträglichkeiten der Wirkstoffe miteinander ist diese Form der Anwendung die bevorzugte Methode.

Im Falle der parenteralen Darreichungsform können die Wirkstoffe auch in Substanz, gegebenenfalls zusammen mit üblichen pharmazeutischen Hilfsstoffen, beispielsweise in lyophilisierter Form vorliegen und durch Zugabe von pharmazeutisch Oblichen Injektionsmedien rekonstituiert oder solubilisiert werden.

Die pharmazeutischen Präparate kommen in flüssiger oder fester Form zur enteralen oder parenteralen Applikation. Hierbei kommen alle üblichen Applikationsformen in Frage, beispielsweise Tabletten, Kapseln, Dragees, Sirupe, Lösungen, Suspensionen. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z.B. Tartrat- und Citratpuffer, Ethanol, Komplexbildner wie z.B. Ethylendiamintetraessigsäure und deren nichttoxischen Salze, sowie hochmolekulare Polymere wie flüssiges Polyethylenoxid zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Kieselsäuren, höhermolekulare Fettsäuren wie Stearinsäure, Gelatine, Agar-Agar, Calciumphosphat, Magnesiumstereat, tierische und pflanzliche Fette, feste hochmolekulare Polymere wie Polyethylenglykole; für orale Applikation geeignete Zubereitungen können gewünschtenfalls Geschmacks- und Süßstoffe enthalten.

Die Dosierung kann von verschiedenen Faktoren wie Applikationsweise, Spezies, Alter und individuellen Zustand abhängen. Die täglich zu verabreichenden Dosen liegen bei 0,005 - 100 mg/kg Körpergewicht pro Einzelkomponente.

Bei den Kombinationspräparaten kann sich das Verhältnis zwischen den Wirkstoffen in einem sehr weiten Bereich bewegen. So sind beispielsweise molare Verhältnisse zwischen 1 : 10 bis 1 : 1000 und 10 : 1 bis 1000 : 1 möglich, je nach Wirksamkeit der in Frage kommenden Wirkstoffe. Im Falle der Kombination mit Cytostatika ist ein Verhältnis zwischen 1 : 100 und 100 : 1 bevorzugt.

In einer besonder bevorzugten Ausführungsform betrifft die vorliegende Erfindung ein pharmazeutisches Kombinationspräparat bestehend aus mindestens einer Verbindung mit Glutaminase-Aktivität und mindestens einem Platin-Komplex, insbesondere cis-Platin. Es wurde festgestellt, dass Platin-Komplexe in der Kombination mit Glutaminase, insbesondere Pseudomonas-Glutaminase, in Gewebekulturen synergistische Effekte an unterschiedlichen Tu-Zelllinien bis zu einem Faktor 120 zeigen. Die Dosis von cis-Platin und Glutaminase kann dadurch jeweils deutlich gegenüber den bei Einzeltherapien benötigten Dosismengen verringert werden. So beträgt die therapeutische Dosis für Glutaminase in den erfindungsgemäßen Kombinationspräparaten vorzugsweise 50 -150 I.U./m$^2$, insbesondere 100 - 130 I.U./m$^2$. Die Dosis an Platin-Komplex, insbesondere cis-Platin, beträgt bei den erfindungsgemäßen Kombinationspräparaten vorzugsweise 1-20 mg/m$^2$, insbesondere 2 - 15 mg/m$^2$ und noch mehr bevorzugt 5 -10 mg/m$^2$ Körperoberfläche. Bei solchen Dosierungen wird bereits ein Ansprechen von soliden Tumoren bei fünftägiger Gabe beobachtet. Bei dreiwöchiger Gabe beträgt die Dosis vorzugsweise 10-100 mg/m$^2$, insbesondere 20 - 50 mg/m$^2$.

Bei Kombinationspräparaten umfassend eine Glutaminase und ein Anthracyclin, insbesondere Doxorubicin, beträgt die Dosis für Glutaminase wiederum bevorzugt 50-150 I.U./m$^2$, insbesondere 100-130 I.U./m$^2$ Körperoberfläche. Die Menge an Doxorubicin beträgt vorteilhafterweise 1 - 20 mg/m$^2$, insbesondere 2-15 mg/m$^2$ und noch mehr bevorzugt 5-10 mg/m$^2$ Körperoberfläche bei ein Mal wöchentlicher Applikation. Bei dreiwöchiger Verabreichung beträgt die bevorzugte Dosis 5-60 mg/m$^2$, insbesondere 10-50 mg/m$^2$ und noch mehr bevorzugt 15-30 mg/m$^2$ Körpergewicht.

Die folgenden Beispiele belegen die synergistische Wirkung einiger repräsentativer Kombinationspräparate.

**Beispiel 1**

In-vitro Tests zur Untersuchung der anti-tumoralen Wirkung von Wirkstoffen

Die anti-tumorale Wirkung von Substanzen wurde in einem in-vitro ZellkulturTest mittels Sulforhodamin-Methode untersucht (Boyd MR, The NCI In Vitro Anticancer Drug Discovery Screen. In: Anticancer Drug Development Guide: Preclinical Screening, Clinical Trials and Approval (Eds. Teicher B. and Totowa N), 1985-1995; Skehan P et al. (1990), "New Clorimetric Assay for AnticancerDrug Screening", J Natl. Can. Instit. 82: 1107-1112). Für den Test wurden Zelllinien aus Tumoren der Brust (MCF7), Lunge (NCI-H460, A549), Colon (SW-60, HT29) und CNS (SF-539) verwendet. Die Kultivierung der Tumorzellen erfolgte im RPMI 1640 Medium mit 7,5 % fötalem Kälberserum bei 37 °C und 5% $CO_2$. Nach Anwachsen der Zellen für 24 Stunden erfolgte die Inkubation mit den zu testenden Substanzen für 48 Stunden. Als Kontrolle dienten Ansätze ohne Wirkstoff, der Nullwert wurde vor Zugabe der Wirkstoffe bestimmt.

Die für die Versuche eingesetzten Antineoplastika wurden von Sigma in Zellkultur-Qualität bezogen.

Als Glutaminase wurde eine mit Polyethylenglykol modifizierte Pseudomonas 7A Glutaminase-Asparaginase (DE 41 40 003 A1, WO 94/13817 A1 und WO 02/31498 A2) verwendet.

**Beispiel 2**

Bestimmung von synergistischen Effekten

Nach der 48-stündigen Inkubationszeit wurden das Zellwachstum mittels Absorption des gebundenen Sulforhodamin-Farbstoffes bei 575 nm bestimmt. Das prozentuale Wachstum wurde wie folgt berechnet:

$$PW = \frac{(T_t - T_0)}{(C - T_0)} \cdot 100$$

wobei

PW prozentuales Wachstum bedeutet,
C für die unbehandelten Kontrollzellen steht,
T die Menge der behandelten Zellen bedeutet

und die Indices

0 und t für die Menge der Zellen zum Zeitpunkt 0 und nach 48 Stunden steht.

**Beispiel 2a**

Kombination aus Mitomycin und Glutaminase

In Abbildung 1 ist die anti-tumorale Wirkung von 0,026 µg/mL Mitomycin auf Zellen eines CNS Tumors (SF-539) und Brust (MCF7) Tumors alleine und in Kombination mit 0,001 U/mL Glutaminase dargestellt. Mitomycin alleine zeigt keine Wirkung auf CNS-Zellen, in Kombination mit Glutaminase wird das Wachstum im Vergleich zur Kontrolle auf 7 % reduziert. Mitomycin reduziert das Wachstum von Zellen des Brusttumors MCF7 auf 66 % im Vergleich zur Kontrolle. Durch Kombination mit 0,001 U/mL Glutaminase wird das Wachstum auf 40 % reduziert.

**Beispiel 2b**

Kombination aus Mitoxantron und Glutaminase

In Abbildung 2 ist die anti-tumorale Wirkung von 0,3 µg/mL Mitoxantron auf Zellen eines Brust (MCF7), Lungen (NCI-H460) und Colon (SW-60) Tumors alleine und in Kombination mit 0,001 U/mL Glutaminase dargestellt. Glutaminase allein zeigt nur geringe Wirkung auf die drei Tumoren. Mitoxantron reduziert das Tumorzell-Wachstum alleine auf 23 % bis 47 %. In Kombination wird das Wachstum von Zellen des Brust-, Lungen- und Colon-Tumors auf 1 %, 9 % bzw. 25 % reduziert.

**Beispiel 2c**

Kombination aus cis-Platin und Glutaminase

In Abbildung 3 ist die anti-tumorale Wirkung von 2 µg/mlL cis-Platin auf Zellen eines Lungen (A549), Brust (MCF7) und Colon (HAT29) Tumors alleine und in Kombination mit 0,001 U/mL Glutaminase dargestellt. cis-Platin allein bewirkt eine Reduktion des Wachstums auf 41 %, 86 % bzw. 65 %. In Kombination mit Glutaminase wird das Wachstum der Tumorzellen auf 15 %, 18 % bzw. 2 % reduziert.

**Beispiel 2d**

Kombination aus Etoposid und Glutaminase

In Abbildung 4 ist die anti-tumorale Wirkung von 2,3 µg/mL Etoposid auf Zellen von Lungen (A549 und NCl-1-123) und Brust (MCF7) Tumoren alleine und in Kombination mit 0,001 U/mL Glutaminase dargestellt.

Etoposid allein reduziert das Wachstum dieser Zellen alleine auf ca. 40 % im Vergleich zur Kontrolle. In Kombination mit Glutaminase wird das Wachstum der Tumorzellen auf 18 % und 6 % bzw. 26 % reduziert.

**Beispiel 2e**

Kombination aus Melphalan und Glutaminase

In Abbildung 5 ist die anti-tumorale Wirkung von 68 µg/mL Melphalan auf Zellen eines Lungen (NCl-H23) Tumors alleine und in Kombination mit 0,001 U/mL Glutaminase dargestellt. Melphalan reduziert das Tumorwachstum auf 34 %. In Kombination mit Glutaminase wird das Wachstum auf 10 % reduziert.

**Patentansprüche**

1. Pharmazeutisches Kombinationspräparat zur Krebstherapie umfassend als Wirkstoffe

   a) mindestens eine Verbindung mit Glutaminase-Aktivität und
   b) mindestens ein Antineoplastikum, ausgewählt aus Platin-Komplexen und Anthracyclinen.

2. Präparat nach Anspruch 1,
   **dadurch gekennzeichnet,**
   **dass** die Verbindung mit Glutaminase-Aktivität eine Glutaminase, GlutaminaseAsparaginase, Glutaminase-Analoga, Derivate oder Modifizierungen dieser sind und entweder natürlichen Ursprungs sind oder synthetisch hergestellt wurden.

3. Präparat nach Anspruch 2,

**dadurch gekennzeichnet,**
**dass** die Verbindung mit Glutaminase-Aktivität aus Pseudomonas ist, vorzugsweise Pseudomonas 7A Glutaminase-Asparaginase.

**4.** Präparat nach einem der Ansprüche 1 bis 3,
**dadurch gekennzeichnet,**
**dass** die Verbindung mit Glutaminase-Aktivität modifiziert ist, vorzugsweise mit Polyethylenglykol.

**5.** Präparat nach einem der Ansprüche 1 bis 4,
**dadurch gekennzeichnet,**
**dass** es Doxorubicin, Daunomycin, Actinomycin D oder/und Mitoxantron umfasst.

**6.** Präparat nach einem der Ansprüche 1 bis 5,
**dadurch gekennzeichnet,**
**dass** es cis-Platin, Oxaliplatin oder/und Carboplatin umfasst.

**7.** Verfahren zur Herstellung von pharmazeutischen Präparaten gemäß einem der Ansprüche 1 bis 6,
**dadurch gekennzeichnet,**
**dass** man die Wirkstoffe gegebenenfalls zusammen mit pharmazeutisch üblichen Träger- oder Hilfsstoffen vermischt und zu oralen oder parenteralen Applikationsformen verarbeitet.

**8.** Verwendung einer Verbindung mit Glutaminase-Aktivität und mindestens einem Antineoplastikum ausgewählt aus Platin-Komplexen und Anthracyclinen zur Herstellung eines Mittels für eine antineoplastische Therapie.

**9.** Verfahren zur Herstellung eines Mittels zur Behandlung von Krebs und anderen Krankheiten, die im Zusammenhang mit abnormaler Zellproliferation stehen,
**dadurch gekennzeichnet,**
**dass** mindestens eine Verbindung mit Glutaminase-Aktivität und mindestens ein Antineoplastikum ausgewählt aus Platin-Komplexen oder Anthracyclinen in einem molaren Verhältnis zwischen 1 : 10 bis 1 : 1000 und 10 : 1 bis 1000 : 1 zu applizieren ist, wobei die täglich zu verabreichenden Dosen bei 0,005 - 100 mg/kg Körpergewicht pro Einzelkomponente liegen.

## Claims

**1.** Combined pharmaceutical preparation for cancer therapy comprising as active substances

a) at least one compound having glutaminase activity and
b) at least one antineoplastic agent selected from platinum complexes and anthracyclines.

**2.** Preparation according to claim 1,
**characterized in that**
the compound having glutaminase activity is a glutaminase, glutaminase-asparaginase, glutaminase analogue, derivative or modification of the same and is either of natural origin or is produced synthetically.

**3.** Preparation according to claim 2,
**characterized in that**
the compound having glutaminase activity is from Pseudomonas and is preferably Pseudomonas 7A glutaminase-asparaginase.

**4.** Preparation according to one of the claims 1 to 3,
**characterized in that**
the compound having glutaminase activity is modified preferably with polyethylene glycol.

**5.** Preparation according to one of the claims 1 to 4,
**characterized in that**
it comprises doxorubicin, daunomycin, actinomycin D or/and mitoxantrone.

**6.** Preparation according to one of the claims 1 to 5,
**characterized in that**
it comprises cis-platinum, oxaliplatinum or/and carboplatinum.

**7.** Process for producing pharmaceutical preparations according to one of the claims 1 to 6,
**characterized in that**
the active substances optionally together with common pharmaceutical carrier substances or auxiliary substances are mixed and processed into oral or parenteral forms of administration.

**8.** Use of a compound having glutaminase activity and at least one antineoplastic agent selected from platinum complexes and anthracyclines to produce an agent for an antineoplastic therapy.

**9.** Process for producing an agent to treat cancer and other diseases which are associated with abnormal cell proliferation,
**characterized in that**
at least one compound having glutaminase activity and at least one antineoplastic agent selected from platinum complexes or anthracyclines are to be administered in a molar ratio between 1:10 to 1:1000 and 10:1 1 to 1000:1, where the doses to be administered daily are 0.005-100 mg/kg body weight per individual component.

## Revendications

**1.** Préparation pharmaceutique combinée pour la thérapie du cancer, comprenant comme substances actives:

a) au moins un composé à activité de glutaminase, et
b) au moins un antinéoplasique choisi parmi des complexes du platine et des anthracyclines.

2. Préparation selon la revendication 1, **caractérisée en ce que** le composé à activité de glutaminase est une glutaminase, une glutaminase-asparaginase, un analogue de glutaminase, un dérivé ou une variante de celles-ci, et est d'origine naturelle ou a été préparé par synthèse.

3. Préparation selon la revendication 2, **caractérisée en ce que** le composé à activité de glutaminase provient d'un *Pseudomonas*, et est de préférence une glutaminase-asparaginase de *Pseudomonas* 7A.

4. Préparation selon l'une des revendications 1 à 3, **caractérisée en ce que** le composé à activité de glutaminase est modifié, de préférence avec du polyéthylèneglycol.

5. Préparation selon l'une des revendications 1 à 4, **caractérisée en ce qu'**elle comprend de la doxorubicine, de la daunomycine, de l'actinomycine D et/ou de la mitoxantrone.

6. Préparation selon l'une des revendications 1 à 5, **caractérisée en ce qu'**elle comprend du cis-platine, de l'oxaliplatine et/ou du carboplatine.

7. Procédé de préparation de préparations pharmaceutiques selon l'une des revendications 1 à 6, **caractérisé en ce que** l'on mélange les substances actives, éventuellement avec des supports ou des auxiliaires classiques en pharmacie et on les transforme en des formes d'administration orales ou parentérales.

8. Utilisation d'un composé à activité de glutaminase et d'au moins un antinéoplasique choisi parmi des complexes du platine et des anthracyclines pour la préparation d'une composition destinée à une thérapie antinéoplasique.

9. Procédé de préparation d'une composition destinée au traitement du cancer et d'autres maladies qui sont liées à une prolifération cellulaire anormale, **caractérisé en ce qu'**il faut appliquer au moins un composé à activité de glutaminase et au moins un antinéoplasique choisi parmi des complexes du platine ou des anthracyclines en un rapport molaire entre 1:10 à 1:1000 et 10:1 à 1000:1, les doses à administrer par jour étant de 0,005-100 mg/kg de masse corporelle par constituant individuel.

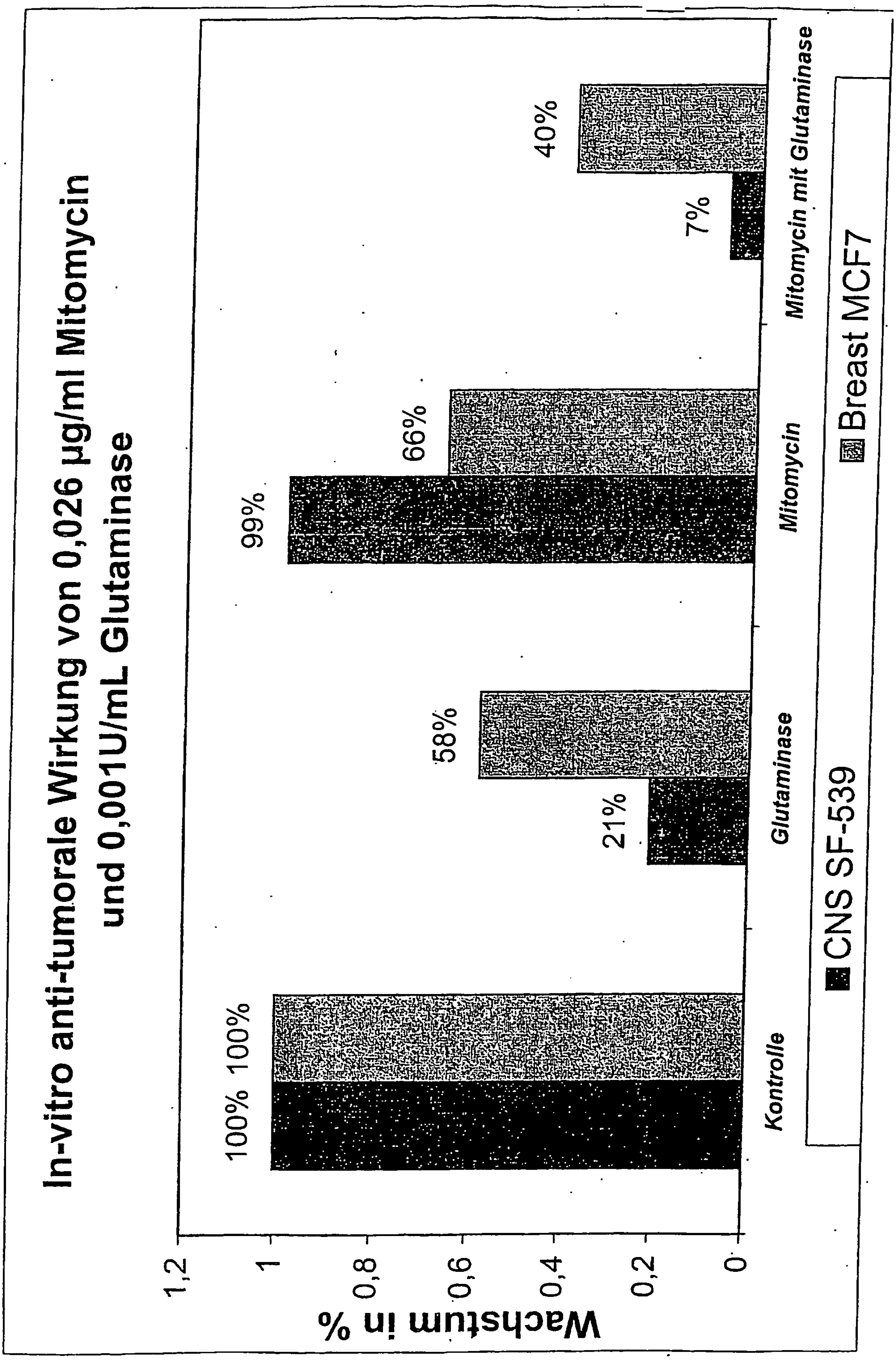
In-vitro anti-tumorale Wirkung von 0,026 µg/ml Mitomycin
und 0,001U/mL Glutaminase
Wachstum in %
1,2
1
0,8
0,6
0,4
0,2
0
100%
100%
21%
58%
99%
66%
7%
40%
Kontrolle
Glutaminase
Mitomycin
Mitomycin mit Glutaminase
CNS SF-539
Breast MCF7

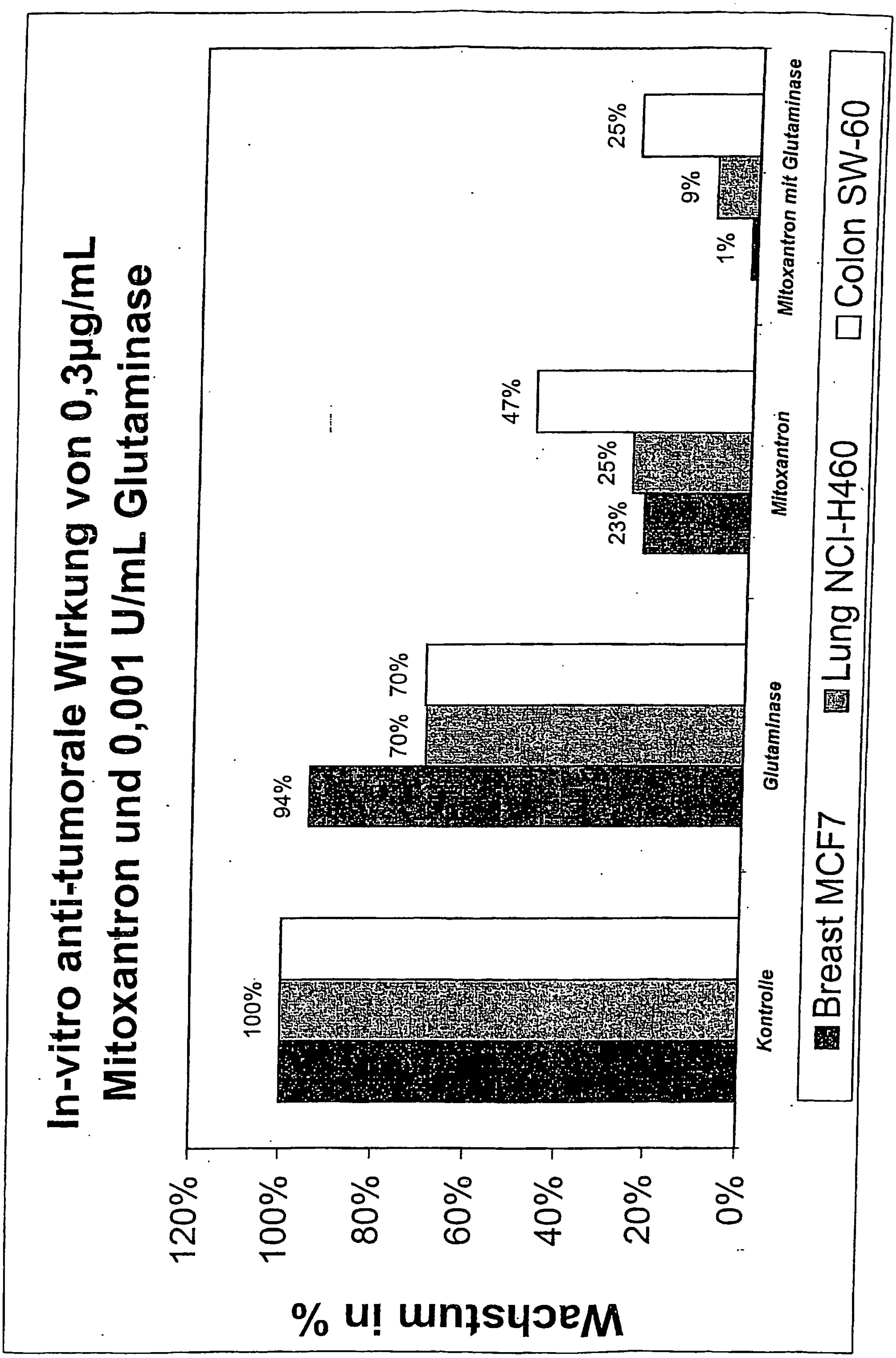
In-vitro anti-tumorale Wirkung von 0,3µg/mL
Mitoxantron und 0,001 U/mL Glutaminase
Wachstum in %
120%
100%
80%
60%
40%
20%
0%
100%
94%
70%
70%
23%
25%
47%
1%
9%
25%
Kontrolle
Glutaminase
Mitoxantron
Mitoxantron mit Glutaminase
Breast MCF7
Lung NCI-H460
Colon SW-60

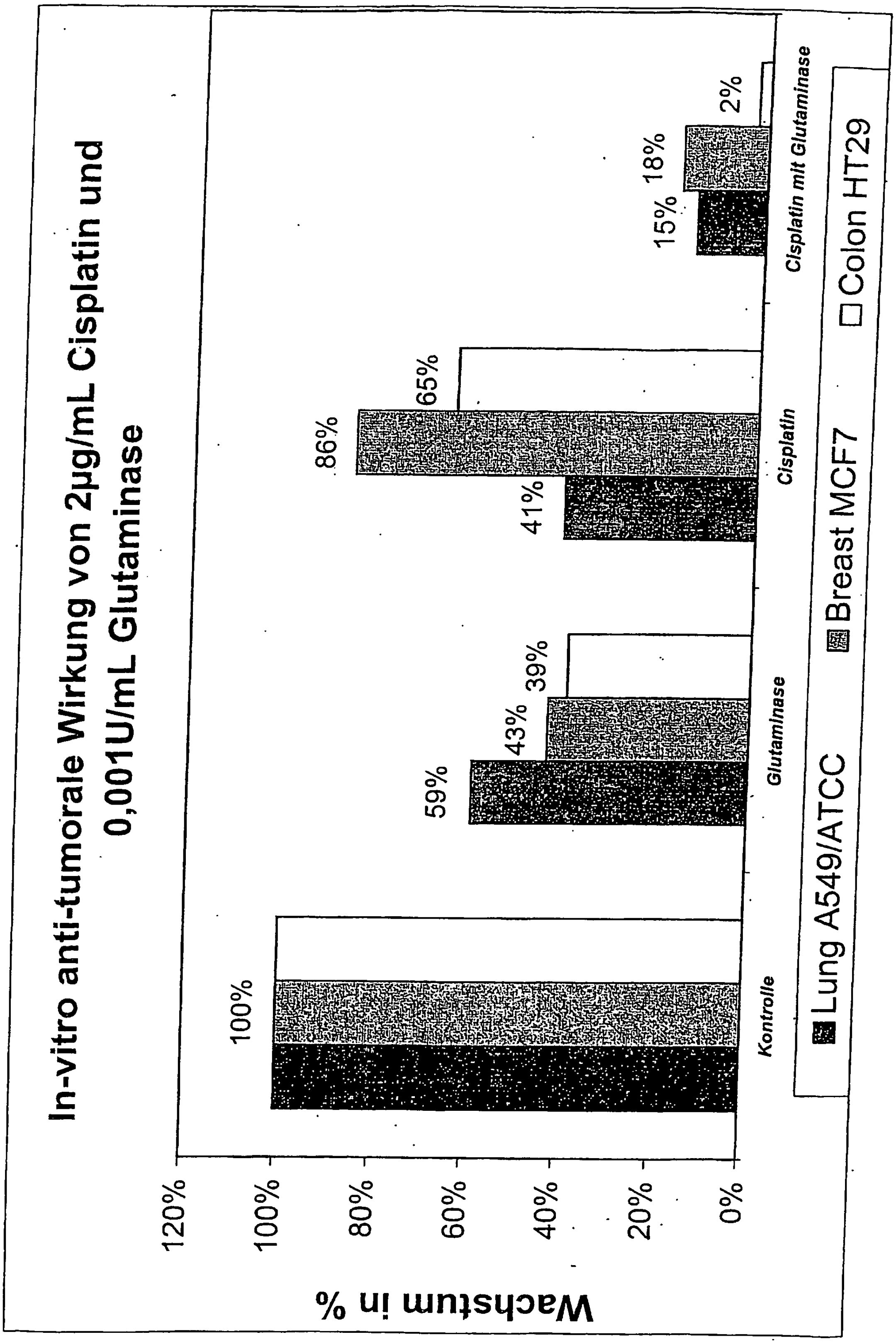
In-vitro anti-tumorale Wirkung von 2µg/mL Cisplatin und 0,001U/mL Glutaminase
Wachstum in %
120%
100%
80%
60%
40%
20%
0%
100%
59%
43%
39%
41%
86%
65%
15%
18%
2%
Kontrolle
Glutaminase
Cisplatin
Cisplatin mit Glutaminase
Lung A549/ATCC
Breast MCF7
Colon HT29

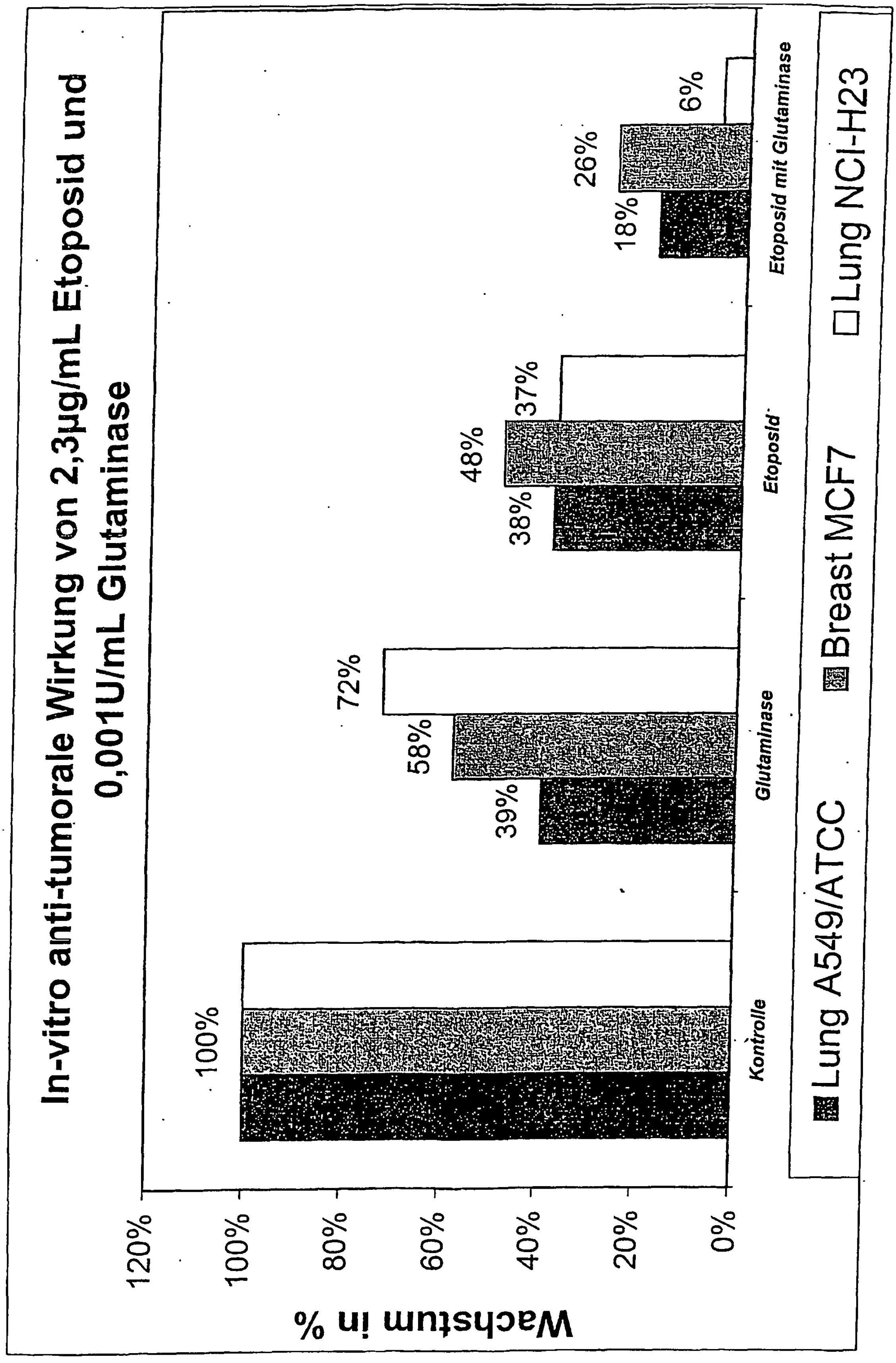
In-vitro anti-tumorale Wirkung von 2,3µg/mL Etoposid und 0,001U/mL Glutaminase
Wachstum in %
120%
100%
80%
60%
40%
20%
0%
100%
39%
58%
72%
38%
48%
37%
18%
26%
6%
Kontrolle
Glutaminase
Etoposid
Etoposid mit Glutaminase
Lung A549/ATCC
Breast MCF7
Lung NCI-H23

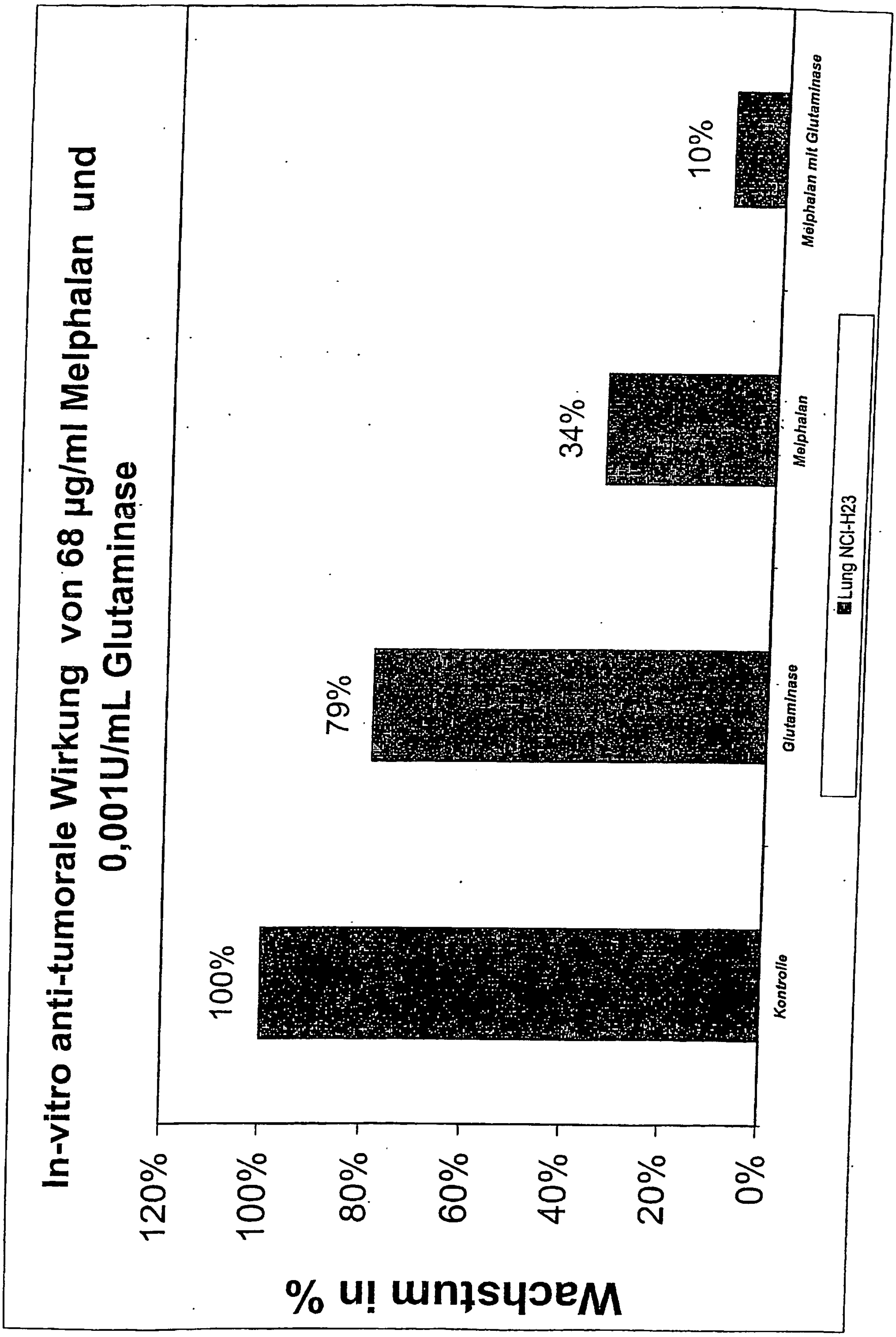
In-vitro anti-tumorale Wirkung von 68 µg/ml Melphalan und 0,001U/mL Glutaminase
Wachstum in %
120%
100%
80%
60%
40%
20%
0%
100%
79%
34%
10%
Kontrolle
Glutaminase
Melphalan
Melphalan mit Glutaminase
Lung NCI-H23